# FASCICULE DE BREVET EUROPEEN

(11) **EP 4 346 458 B1**
(45) Date de publication et mention de la délivrance du brevet: **25.06.2025**
(21) Numéro de dépôt: 22732298.9
(22) Date de dépôt: 24.05.2022
(51) Int. Cl.: A24D 3/06, A24D 3/14

(54) **FORMULATION DE MICROPARTICULES A BASE DE COMPOSES POLYPHENOLIQUES CAPABLES DE PIEGER LES RADICAUX LIBRES PRESENTS DANS L'AIR POLLUE ET LES FUMEES**
FORMULIERUNG VON MIKROPARTIKELN AUF BASIS VON POLYPHENOLISCHEN VERBINDUNGEN ZUR ENTFERNUNG VON FREIEN RADIKALEN IN VERUNREINIGTER LUFT UND IM RAUCH
FORMULATION OF MICROPARTICLES BASED ON POLYPHENOLIC COMPOUNDS CAPABLE OF SCAVENGING FREE RADICALS PRESENT IN POLLUTED AIR AND IN SMOKE

(30) Priorité: 24.05.2021 FR 2105378
(43) Date de publication de la demande: 10.04.2024
(73) Titulaire: Emami, Iman, 92320 Chatillon (FR)
(72) Inventeur: Emami, Iman, 92320 Chatillon (FR)
(74) Mandataire: Lapienis, Juozas
(86) Numéro de dépôt international: PCT/FR2022/050990
(87) Numéro de publication internationale: WO 2022/248803

(56) Documents cités:
- EP-A1- 1 611 807
- CN-B- 108 338 406
- FR-A1- 2 564 296
- FR-A1- 2 772 561
- JP-A- 2003 102 457
- MA-B1- 30 997

## Description

L'invention se rapporte au domaine des capteurs de radicaux libres. Plus particulièrement, l'invention concerne des microparticules associées à des composés polyphénoliques capables de capter des radicaux libres présents dans l'air pollué ou les fumées, notamment des fumées de cigarettes à base de tabac, de CBD et/ou de cannabis ou leurs mélanges.

### Domaine de l'invention

La présente invention a trait à l'utilisation de composés polyphénoliques ou de leurs dérivés comme capteurs de radicaux libres dans des dispositifs destinés à réduire les effets nocifs de la pollution d'air, de la fumée générée par la combustion de tabac, de la fumée générée par la combustion de cannabis (on considère ici le cannabis à usage médical), ou de la fumée générée par la combustion de CBD ou un mélange de ces fumées, ou un mélange de ces fumées et la pollution d'air. Une telle utilisation est décrite dans le brevet EP1041899B1.

De nombreux composés polyphénoliques sont connus pour leurs propriétés bénéfiques dans des domaines aussi variés que l'hypertension, l'hypercholestérolémie, les maladies cardiovasculaires, les infections virales, ou encore les phénomènes inflammatoires. Les activités anti-lipoperoxydantes et anti-carcinogènes de certains polyphénols ont également été décrites.

Il a notamment été montré que l'épigallocatéchine-3-gallate (EGCG), le principal polyphénol présent dans le thé vert, possède une puissante activité anti-inflammatoire et antiproliférative capable d'inhiber sélectivement la croissance cellulaire et d'induire l'apoptose dans les cellules cancéreuses sans nuire aux cellules normales (D N Syed et al. Green tea polyphenol EGCG suppresses cigarette smoke condensate-induced NF-κB activation in normal human bronchial epithelial cells Oncogene Volume 26, pages673-682 (2007) doi.org/10.1038/sj.onc.1209829).

Toutefois, à ce jour, l'utilisation de composés polyphénoliques comme moyen de capter les radicaux libres présents dans les fumées de cigarettes et plus généralement dans l'air, et susceptibles d'être inhalés, a été très peu étudiée et doit être améliorée pour être exploitable au niveau industriel. CN 108 338 406 B décrit des perles antioxydantes constituées d'antioxydant liposoluble et soluble de romarin et comprend notamment des huiles essentielles et des huiles solubles.

### Exposé de l'invention

Les auteurs de la présente invention ont à présent découvert que l'incorporation de polyphénols ou de leurs dérivés dans des microparticules permet d'éliminer efficacement les radicaux libres des molécules cytotoxiques de la fumée issue du tabac, de cannabis (à visée médicale), de CBD, de pollution d'air ou de leurs mélanges. Ces microparticules sont incorporées dans un filtre pour cigarette ou bien sur un tissu de type non tissé (de type extrudé soufflé, « meltblown »). Le mélange des composées polyphénoliques formulées dans des microparticules permet d'absorber sélectivement les radicaux libres de la phase gazeuse et la phase semi-liquide (goudrons) lors de leur passage à travers le filtre ou à travers le tissu filtrant.

La présente invention a donc pour objet l'utilisation de composés polyphénoliques formulées dans des microparticules comme capteurs ("scavengers") de radicaux libres. Elle concerne en particulier une formulation de microparticules associées à des composés polyphénoliques et leurs dérivés notamment, sous la forme d'un extrait de romarin, dans laquelle les microparticules sont constituées d'un mélange de 2 à 4 huiles végétales saturées dont les températures de fusion diffèrent d'au moins 10°C.

Cette formulation peut être utilisée comme capteurs de radicaux libres dans les filtres de cigarettes à base de tabac, filtres de cigarettes à base de CBD, filtres de cigarettes à base de cannabis, ou filtres de cigarettes à base de leur mélange et tissu filtrant d'air, notamment dans un masque.

L'invention concerne aussi un procédé de préparation d'un filtre pour cigarette et le filtre obtenu, ainsi qu'un procédé de préparation d'un tissu filtrant et le tissu obtenu.

### Avantages de l'invention

La formulation des composés polyphénoliques selon l'invention présente deux avantages principaux.

D'une part, cette formulation à base d'au moins 2 huiles végétales permet de créer un effet de surface qui optimise l'efficacité anti-radicalaire des composés polyphénoliques. Après chauffage du mélange huiles/ composés polyphénoliques et refroidissement, les composés polyphénoliques se trouvent repoussés vers l'extérieur de la particule et sont ainsi exposés, au contact de l'air, de sorte à pouvoir interagir avec les radicaux libres. Ces composés phénoliques captent les radicaux libres, notamment les radicaux OH°.

Cette composition a un effet de neutralisation de l'effet des goudrons du fait des contacts qui s'établissent à la surface des particules. Dans le cas des fumées de cigarette, les goudrons entrent en compétition avec les composés phénoliques, pour interagir avec les radicaux libres. Ces radicaux libres en phase semi-liquide sont également piégés par les composés polyphénoliques comme ceux en phase gazeuse ; cet effet a en particulier été démontré avec un extrait de romarin riche en polyphénols.

D'autre part, cette approche fournit des particules dont la taille permet d'éviter les risques d'ingestion, que ce soit celle des particules présentes dans le filtre ou sur les tissus filtrants. A cet effet, la taille de particules est adaptée à l'application choisie. Les particules doivent être suffisamment grandes pour être retenues sur leur support (filtre ou tissu), tout en considérant les contraintes liées à la préparation des produits et à la température d'utilisation.

Les filtres à cigarette peuvent être utilisés pour la consommation de tabac et/ou de cannabis, notamment dans le cadre d'utilisation thérapeutique, ou de CBD pour capter les radicaux libres et contrer ainsi leurs effets neurodégénératifs.

Les tissus filtrants sont particulièrement adaptés pour faire des masques anti-pollution pouvant être utilisés dans tout environnement pollué par des particules fines tels que les goudrons, notamment chez les asthmatiques, les cyclistes et sportifs en milieu urbain.

### DESCRIPTION DETAILLEE DE L'INVENTION

Un premier objet de l'invention concerne la formulation de microparticules associées à des composés polyphénoliques et leurs dérivés notamment sous la forme d'un extrait de romarin dans laquelle les microparticules sont constituées d'un mélange de 2 à 4 huiles végétales saturées dont les températures de fusion diffèrent d'au moins 10°C.

Par "dérivés", on entend notamment les composés dérivant des composés polyphénoliques par substitution de l'atome d'hydrogène d'au moins un des groupements hydroxy des composés polyphénoliques par un groupement alkyle en C1-C6, ou un groupement (alkyl en C1-C4)carbonyle. Les acétates tels que les acétates de l'acide carnosique et les acétates de l'acide rosmarinique sont des dérivés préférés des composés polyphénoliques utilisés conformément à l'invention.

D'autre part, les dérivés de polyphénols utilisés conformément à l'invention tels que le carnosol, le rosmanol, l'acide rosmarinique, l'acide carnosique peuvent correspondre aux isomères desdits polyphénols tels que notamment l'épirosmanol et l'isorosmanol (Nakatani et al., Agric. Biol. Chem., 1984, vol. 48, n° 8, pp 2081-2085, https://doi.org/10.1080/00021369.1984.10866436).

Ces composés peuvent être obtenus par synthèse chimique classique ou par voie biotechnologique, selon des procédés connus de l'homme du métier. Ils peuvent également être isolés d'extraits végétaux.

Dans un mode de réalisation préféré de l'invention, les polyphénols utilisées sont apportés sous la forme d'un extrait de romarin (*Rosmarinus officinalis L*.). Un tel extrait végétal peut être obtenu par extraction par un solvant polaire tel qu'un solvant alcoolique ou hydro-alcoolique. L'alcool utilisé comme solvant peut être notamment l'éthanol. Cet extrait peut également être avantageusement obtenu à l'aide de dioxyde de carbone supercritique et est alors plus riche en composés polyphénoliques. Un tel extrait végétal peut être obtenu par extraction par un solvant polaire, un solvant apolaire, un mélange de solvants polaire et apolaire, à l'aide de dioxyde de carbone supercritique ou l'utilisation successives de toutes ces méthodes.

De manière préférée, l'extrait végétal utilisé selon l'invention est obtenu par extraction par un solvant polaire suivie par une extraction par un solvant apolaire et suivie par une extraction au CO₂ supercritique.

L'extraction de romarin est réalisée de préférence sur des plantes séchées, par exemple sur des branches de romarin, coupées et séchées au soleil pendant 4 à 5 jours ou des résidus de distillation pauvre en contenu d'huile essentielle.

Les composés polyphénoliques ou leurs dérivés, obtenus par synthèse chimique, par voie biotechnologique, ou par extraction à partir de végétaux, peuvent être utilisés seuls ou en mélange conformément à l'invention.

Les composés polyphénoliques ou leurs dérivés peuvent être utilisés sous forme libre ou peuvent être conjugués ou couplés à un support ("carrier") permettant de favoriser et amplifier l'action absorbante de l'ensemble polyphénols-support.

Par « huile végétale saturée », on entend toute huile végétale saturée connue de l'homme du métier et notamment l'huile de palme hydrogénée, l'huile de ricin hydrogénée et la cutina, etc...

Dans un mode de réalisation particulier, les composés polyphénoliques sont choisis parmi le carnosol, le rosmanol, l'acide rosmarinique, l'acide carnosique, et leurs dérivés.

Dans un autre mode de réalisation particulier, les composés polyphénoliques sont constitués d'un mélange de camosol, d'acide carnosique et d'acide rosmarinique ou de leurs dérivés.

Dans un autre mode de réalisation particulier, les composés polyphénoliques sont constitués d'un mélange composé en tout ou partie de carnosol.

Dans un autre mode de réalisation particulier, les composés polyphénoliques sont constitués d'un mélange composé en tout ou partie d'acide carnosique.

Dans un mode de réalisation préféré de l'invention, lesdits composés polyphénoliques sont apportés par un extrait de romarin purifié à au moins 60%.

Le ratio en poids entre les composés polyphénoliques et le mélange d'huiles est compris entre 25:75 et 65 :35.

Dans le cadre de la présente invention, la différence de température de fusion entre les huiles est d'au moins 10°C, et peut être d'au moins 15°C.

Un deuxième objet de l'invention concerne l'utilisation d'une formulation telle que définie précédemment comme capteurs de radicaux libres dans les filtres de cigarettes à base de tabac, filtres cigarettes à base de CBD, filtres de cigarettes à base de cannabis, ou filtres de cigarettes à base de leur mélange et dans un tissu filtrant d'air, notamment dans un masque.

Les microparticules se comportent de manière « ingénieuse ». En effet, elles présentent une surface de contact démultipliée pour la réaction d'oxydoréduction, ce qui contribue à l'élimination des radicaux libres. L'augmentation de l'enthalpie libre de la réaction entraine une accélération du processus d'adduction des radicaux en phase gazeuse à des microparticules comprenant des composés polyphénoliques au lieu de leur adduction à des particules de goudron. Il en résulte une réduction de 30% à 65% des radicaux des goudrons (phase semi liquide) notamment des semi-quinones rendant ainsi les goudrons moins oxydants et moins mutagènes. On peut parler ici d'un effet de neutralisation des goudrons.

Ainsi, les microparticules réduisent l'effet mutagène et cancérigènes des fumées et ralentissent l'effet de raidissement qui s'opère au niveau des artères de fait de la présence de composés oxydants.

Un troisième objet de l'invention concerne un procédé de préparation d'un filtre pour cigarette capable de capter les radicaux libres, notamment les radicaux libres présents dans les fumées, consistant à :
- mélanger des microparticules formées d'un mélanges de 2 à 4 huiles végétales saturées, dont les températures de fusion diffèrent d'au moins 10°C, avec des composés polyphénoliques ou leurs dérivés, notamment sous la forme d'un extrait purifié de romarin, et
- incorporer lesdites microparticules dans un filtre pour cigarettes à base de tabac, filtre pour cigarettes de CBD, filtre pour cigarettes de cannabis ou filtre pour cigarettes avec une densité de mélange de 0,3 mg/mm³ à 1 mg/mm³.

Un quatrième objet de l'invention concerne un filtre pour cigarette obtenu par le procédé décrit précédemment dans lequel les microparticules ont une taille comprise entre 70-200 microns et le filtre obtenu présente une résistance au tirage inférieure à 1200 mm d'eau.

Dans un mode de réalisation particulier, les microparticules sont incorporées dans la partie du filtre située du côté de la substance à consommer sur une longueur de 3mm à 10mm. De manière préférée, la partie du filtre contenant les microparticules se situe dans une zone de 6mm de long, située à 3mm de l'extrémité interne du filtre (coté tabac ou autre substance à consommer). Cette disposition permet d'une part d'éviter le contact direct des microparticules avec une chaleur excessive qui les ferait fondre et d'autre part de retenir mécaniquement les microparticules pour qu'elles ne migrent pas vers la bouche, évitant leurs ingestions.

L'usage d'un tel filtre permet une baisse des radicaux libres dans la phase gazeuse, une baisse des radicaux libres dans la phase semi liquide, avec pour conséquence chez le fumeur une baisse du stress oxydatif, une baisse de l'effet oxydant de la fumée, une baisse des effets inflammatoires, une réduction de vieillissement accéléré, une baisse de la réduction de risques d'exposition aux maladies : AVC, neurodégénératifs, crise cardiaque, cancer, etc...

Un cinquième objet de l'invention concerne un procédé de préparation d'un tissu filtrant capable de capter les radicaux libres, notamment les radicaux libres présents dans les fumées et l'air pollué, consistant à :
- mélanger des microparticules formées de mélanges de 2 à 4 huiles végétales saturées, dont les températures de fusion diffèrent d'au moins 10°C, avec des composés polyphénoliques ou leurs dérivés, notamment sous la forme d'un extrait purifié de romarin et
- déposer ces microparticules sur la surface d'un tissu filtrant (de type extrudé soufflé, « meltblown ») avec une densité de 15 gsm (gram per square meter/gramme par mètre carré) à 80 gsm.

Un sixième objet de l'invention concerne un tissu filtrant obtenu par le procédé décrit précédemment dans lequel lesdites microparticules ont une taille comprise entre 1 à 20 microns et une résistance au tirage inférieure à 150 % du standard FFP2, FFP3 ou N95.

Ce tissu filtrant est particulièrement adapté à la préparation d'un masque pour capter les radicaux libres présents dans l'air pollué. Dans ce cas, les microparticules sont appliquées sur la face externe au moins l'une des couches en « meltblown » (tissu extrudé soufflé), de préférence au moins sur la couche de meltblown la plus externe du masque. Ce type de masque est adapté en particulier aux asthmatiques et sportifs ou usager en milieu pollué comme peut l'être le milieu urbain.

L'absorption des radicaux libres et la réduction induite de l'impact des molécules cytotoxiques (voir Alexandrov, K., Rojas, M., & Rolando, C. (2006). DNA damage by benzo (a) pyrene in human cells is increased by cigarette smoke and decreased by a filter containing rosemary extract, which lowers free radicals. Cancer research, 66(24), 11938-11945. DOI: 10.1158/0008-5472.CAN-06-3277) favorisent la réduction du stress oxydatif, la réduction des maladies cardiovasculaires, la réduction du taux de mutagénèse dans les tissus cellulaires, la réduction de l'oxydation des lipides, la réduction des maladies neuro-dégénératives, la réduction du risque des accidents cardiovasculaires, la réduction de la fréquence des crises d'asthme notamment durant les pics de pollution, la réduction du risque de cancers chez les fumeurs, notamment du cancer des poumons et des voies inférieures, la réduction du risque des cancers des testicules, la réduction des inflammations de la bouche, la réduction des inflammations de la langue et des voies supérieures et la réduction des phénomènes inflammatoires.

La présente invention sera mieux comprise à la lecture des exemples qui suivent, fournis à titre d'illustration et ne devant en aucun cas être considérés comme limitant la portée de la présente invention.

### DESCRIPTION DES FIGURES

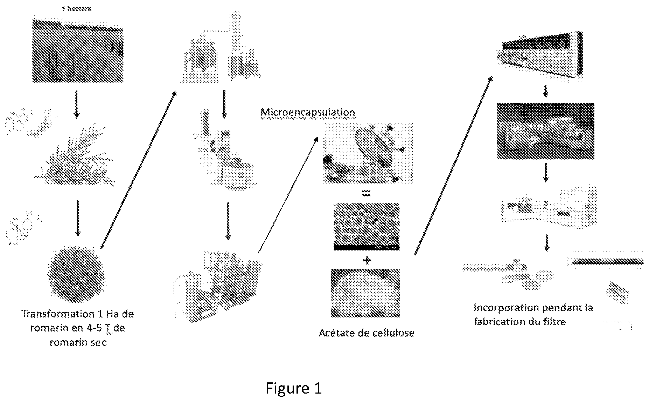

**Figure 1** **:** Vue schématique du procédé de préparation d'un filtre contenant un extrait de romarin

### EXEMPLES

### EXEMPLE 1 : Préparation d'un extrait de romarin

Des épis de romarin (*Rosmarinus officinalis L*.) sont soumis à une extraction par de l'éthanol, à 65°C. Le volume d'éthanol utilisé (en litres) correspond à cinq fois le poids en kg des épis de romarin.

L'extrait est ensuite purifié et enrichi en polyphénols par extraction sélective au CO2 supercritique. Selon le réglage de la température entre 40°C et 100°C et de la pression entre 1 à 170 bars, l'extrait est purifié et enrichi de façon sélective en ses différents composants pour atteindre une pureté d'acide carnosic et caronsol supérieures à 50%.

Un lavage avec un ou plusieurs solvants permet de réduire notablement l'odeur de l'extrait à 50% tout en augmentant la concentration d'acide carnosic et caronsol à 65% ou plus.

Un tel extrait contient les composés suivants : carnosol, rosmanol, rosmadial, acide carnosique, genkwanine, acide rosmarinique...

Les proportions de ces différents composants varient selon le plant de romarin utilisé. Généralement, on obtient un extrait comprenant environ 10% à 25 % d'acide rosmarinique, environ 40% à 50% d'acide carnosique et environ 5% à 10% de carnosol.

### EXEMPLE 2 : Incorporation de mélange dans un filtre de cigarette ou dans un tissu filtrant.

Un filtre de cigarette est préparé par incorporation des microparticules sur la surface de l'acétate de cellulose ou dans un papier crêpé lors de son enroulement. Il est préféré que la partie du filtre contenant les microparticules se situe dans une zone de 6mm de long, située à 3mm de l'extrémité interne du filtre (coté tabac ou autre substance à consommer).

Le tissu filtrant est préparé par épandage des particules sur la surface de tissu et grâce à une charge électrostatique selon le procédé connu par l'homme de métier.

### EXEMPLE 3 : Méthode d'évaluation de l'efficacité du filtre ou de tissu contenant le mélange de microparticules et nanoparticules et de polyphénols et leurs dérivés.

### 1. Modélisation assistée par ordinateur

L'efficacité du filtre de cigarette ou de tissu filtrant dans le captage de radicaux libres ainsi préparé est mise en évidence dans un premier temps par modélisation assistée par ordinateur, selon la méthode de Monte-Carlo, qui permet de calculer le nombre de rencontres entre une molécule cible carcinogène et un composé polyphénolique utilisé conformément à l'invention.

Le quantité de molécules cytotoxiques à radicaux libres présents dans la fumée de cigarette a été calculée de part et d'autre du filtre.

La quantité de molécules cytotoxiques comportant des radicaux libres présents dans l'air traversant le tissu filtrant a été calculée de part et d'autre par cm² de surface de tissu filtrant.

Le nombre de molécules cytotoxiques après la filtration est fonction du volume de fumée passant par le filtre, de la surface du filtre, de la concentration en molécules cytotoxiques dans la fumée et de la concentration de particules issues de mélange d'huiles végétales saturées et de polyphénols dans le filtre.

Les auteurs de la présente invention ont ainsi montré que 10 mg de particules dans le filtre d'une cigarette permet de réduire de plus de 60 % le taux de molécules à radicaux libres cytotoxiques dans la fumée de cigarette à base de tabac, CBD ou cannabis.

### 2. Dosage quantitatif par spectrométrie de masse de la filtration des radicaux libres de la fumée de cigarette

Le dosage quantitatif par spectrométrie de masse de la filtration des radicaux libres est effectué en couplage chromatographie liquide (Liquid chromatography, LC) spectrométrie de masse en tandem (LC / MS-MS) en mode de surveillance de réactions multiples Multiple Reaction Monitoring, MRM) pour quantifier la teneur en radicaux libres OH° dans la fumée de cigarette. Ce mode permet d'avoir une analyse plus spécifique et plus sensible. En effet, le spectromètre ne détecte que quelques transitions plutôt que tout un spectre de masse, d'où un rapport signal sur bruit plus élevé. Le piège à spin 4,5,5-triméthyl-pyrroline-N-oxyde (TMPO) a été utilisé dans le benzène. L'expérience a été réalisée en spécifiant la masse de l'ion parent et en surveillant sa transition. Un piège à spin du commerce, le 3-amino-2,2,5,5-tétraméthyl-1-pyrrolidinyloxyde, a été utilisé comme étalon interne pour la quantification des radicaux libres. La quantité absolue de radicaux libres mesurée ici était en bon accord avec les expériences ESR.

Les résultats sont présentés dans le Tableau 1 ci-dessous.

**Tableau 1: Résultat comparaison du dosage quantitatif par spectrométrie de masse de la filtration des radicaux libres de la fumée de cigarette par des filtres duaux incorporés : la première référence est sans additif. Le filtre A est doté de 15 mg d'extrait de romarin réparti sur une longueur de 6mm. Le filtre B est doté de 15 mg de particules issus de mélanges tel que précédemment exposé d'extrait de romarin et d'huiles végétales saturées réparti sur une longueur de 6mm. Les trois filtres sont à base d'acétate de cellulose.**

| | **Référence** | **Filtre A** | **Filtre B** |
|---|---|---|---|
| Longueur de dépôt 6 mm et de filtre | 0,27 | 6,27 | 6,27 |
| Extrait de romarin, ou particules en poids (mg) déposé | - | 15 | 15 |
| Perte de pression (mm H₂O) | | 86 | 86 |
| Bouffées pour 5 cigarettes | 72 | 74 | 74 |
| Nombre de radicaux libres OH°×10⁻¹⁵ | 9,2 | 6,8 | 1,5 |
| Efficacité de piégeage (%) des radicaux libres | - | 26 | 85 |

### 3. Dosage quantitatif par spectrométrie de masse de la filtration des radicaux libres à travers un tissu filtrant

Pour tester l'effet anti-radicalaire associé à l'incorporation de microparticules selon l'invention dans un tissu filtrant, 25 gsm de microparticules de 5 microns ont été déposés sur la surface extérieures d'un meltblown.

La méthodologie d'analyse utilisée est la même que celle décrite précédemment pour les filtres.

Les résultats montrent que la présence de microparticules permet de capter au moins 70% des radicaux libres OH°.

## Revendications

1. Formulation de microparticules associées à des composés polyphénoliques et/ou leurs dérivés, notamment sous la forme d'un extrait de romarin, dans laquelle les microparticules sont constituées d'un mélange de 2 à 4 huiles végétales saturées dont les températures de fusion diffèrent d'au moins 10°C.

2. Formulation selon la revendication 1 dans laquelle lesdits composés polyphénoliques sont choisis parmi le carnosol, le rosmanol, l'acide rosmarinique, l'acide carnosique, et leurs dérivés.

3. Formulation selon la revendication 1 dans laquelle lesdits composés polyphénoliques sont constitués d'un mélange de camosol, d'acide carnosique et d'acide rosmarinique et/ou de leurs dérivés.

4. Formulation selon la revendication 1 dans laquelle lesdits composés polyphénoliques sont constitués d'un mélange composé en tout ou partie de carnosol.

5. Formulation selon la revendication 1 dans laquelle lesdits composés polyphénoliques sont constitués d'un mélange composé en tout ou partie d'acide carnosique.

6. Formulation selon la revendication 1 dans laquelle lesdits composés polyphénoliques sont apportés par un extrait de romarin purifié à au moins 60%.

7. Utilisation d'une formulation telle que définie à l'une des revendications 1 à 6 comme capteurs de radicaux libres dans des filtres de cigarettes à base de tabac, filtres cigarettes à base de CBD, filtres de cigarettes à base de cannabis, ou filtres de cigarettes à base de leur mélange et dans un tissu filtrant d'air, notamment dans un masque.

8. Procédé de préparation d'un filtre pour cigarette capable de capter les radicaux libres, notamment les radicaux libres présents dans les fumées, consistant à :
- mélanger des microparticules formées d'un mélange de 2 à 4 huiles végétales saturées, dont les températures de fusion diffèrent d'au moins 10°C, avec des composés polyphénoliques ou leurs dérivés, notamment sous la forme d'un extrait purifié de romarin et
- incorporer ces microparticules dans un filtre pour cigarettes à base de tabac, filtre pour cigarettes de CBD, filtre pour cigarettes de cannabis ou filtre pour cigarettes avec une densité de mélange de 0,3 mg/mm³ à 1 mg/mm³.

9. Filtre pour cigarette obtenu par le procédé selon la revendication 8 dans lequel lesdites microparticules ont une taille comprise entre 70-200 microns et le filtre présente une résistance au tirage inférieure à 1200 mm d'eau.

10. Procédé de préparation d'un tissu filtrant capable de capter les radicaux libres, notamment les radicaux libres présents dans les fumées et l'air pollué, consistant à :
- mélanger des microparticules formées d'un mélange de 2 à 4 huiles végétales saturées, dont les températures de fusion diffèrent d'au moins 10°C, avec des composés polyphénoliques ou leurs dérivés, notamment sous la forme d'un extrait purifié de romarin et
- déposer ces microparticules sur la surface d'un tissu filtrant avec une densité de 15 gsm à 80 gsm.

11. Tissu filtrant obtenu par le procédé selon la revendication 10 dans lequel lesdites microparticules ont une taille comprise entre 1 à 20 microns et une résistance au tirage inférieure à 150 % du standard FFP2 ou N95 ou FFP3.

## Patentansprüche

1. Rezeptur der Mikropartikel, die mit Polyphenolverbindungen und/oder deren Derivaten assoziiert sind, insbesondere in Form von Rosmarinextrakt, wobei die Mikropartikel aus einer Mischung von 2 bis 4 gesättigten Pflanzenölen bestehen, deren Schmelztemperaturen sich um mindestens 10°C unterscheiden.

2. Rezeptur nach Anspruch 1, wobei die Polyphenolverbindungen aus Carnosol, Rosmanol, Rosmarinsäure, Carnosinsäure und Derivaten davon ausgewählt sind.

3. Rezeptur nach Anspruch 1, wobei die Polyphenolverbindungen aus einer Mischung von Carnosol, Carnosinsäure und Rosmarinsäure und/oder Derivaten davon bestehen.

4. Rezeptur nach Anspruch 1, wobei die Polyphenolverbindungen aus einer Mischung bestehen, die ganz oder teilweise aus Carnosol zusammengesetzt ist.

5. Rezeptur nach Anspruch 1, wobei die Polyphenolverbindungen aus einer Mischung bestehen, die ganz oder teilweise aus Carnosinsäure zusammengesetzt ist.

6. Rezeptur nach Anspruch 1, wobei die Polyphenolverbindungen durch einen auf mindestens 60% gereinigten Rosmarinextrakt bereitgestellt sind.

7. Verwendung einer Rezeptur gemäß Anspruch 1 als Fänger von freien Radikalen in tabakbasierten Zigarettenfiltern, CBD-basierten Zigarettenfiltern, Cannabis-basierten Zigarettenfiltern oder Zigarettenfiltern auf Basis einer Mischung davon und in einem Luftfiltergewebe, insbesondere in einer Maske.

8. Verfahren zur Herstellung eines Zigarettenfilters, der freie Radikale, insbesondere in Rauch vorhandene freie Radikale einfangen kann, umfassend:
Mischen von Mikropartikeln, die aus einer Mischung von 2 bis 4 gesättigten Pflanzenölen, deren Schmelztemperaturen sich um mindestens 10°C unterscheiden, gebildet sind, mit Polyphenolverbindungen oder deren Derivaten, insbesondere in Form eines gereinigten Rosmarinextrakts, und
Einbindung dieser Mikropartikel in einen Filter für Tabakzigaretten, Filter für CBD-Zigaretten, Filter für Cannabis-Zigaretten oder Filter für Zigaretten mit einer Mischungsdichte von 0,3 mg/mm³ bis 1 mg/mm³.

9. Zigarettenfilter, der durch das Verfahren nach Anspruch 8 erhalten wird, wobei die Mikropartikel eine Größe zwischen 70-200 Mikrometern haben und der Filter einen Druckabfall von weniger als 1200 mm Wassersäule hat.

10. Verfahren zur Herstellung eines Filtergewebes, das freie Radikale, insbesondere in Rauch und verschmutzter Luft vorhandene freie Radikale einfangen kann, umfassend
Mischen von Mikropartikeln, die aus einer Mischung von 2 bis 4 gesättigten Pflanzenölen, deren Schmelztemperaturen sich um mindestens 10°C unterscheiden, gebildet sind, mit Polyphenolverbindungen oder deren Derivaten, insbesondere in Form eines gereinigten Rosmarinextrakts, und
Ablagerung dieser Mikropartikel auf der Oberfläche eines Filtergewebes mit einer Dichte von 15 g/m² bis 80 g/m².

11. Filtergewebe, das durch das Verfahren nach Anspruch 10 erhalten wird, wobei die Mikropartikel eine Größe zwischen 1 und 20 Mikrometern und einen Zugwiderstand von weniger als 150% des FFP2- oder N95- oder FFP3-Standards haben.

## Claims

1. Formulation of microparticles associated with polyphenolic compounds and/or their derivatives, especially in the form of rosemary extract, wherein the microparticles consist of a mixture of 2 to 4 saturated vegetable oils whose melting temperatures differ by at least 10° C.

2. Formulation according to claim 1 wherein said polyphenolic compounds are selected from carnosol, rosmanol, rosmarinic acid, carnosic acid, and derivatives thereof.

3. A formulation according to claim 1 wherein said polyphenolic compounds consist of a mixture of carnosol, carnosic acid and rosmarinic acid and/or derivatives thereof.

4. A formulation according to claim 1 wherein said polyphenolic compounds consist of a mixture composed wholly or partly of carnosol.

5. A formulation according to claim 1 wherein said polyphenolic compounds consist of a mixture composed wholly or partly of carnosic acid.

6. A formulation according to claim 1 in which said polyphenolic compounds are provided by a rosemary extract purified to at least 60%.

7. Use of a formulation as defined in claim 1 as a free radical scavenger in tobacco-based cigarette filters, CBD- based cigarette filters, cannabis-based cigarette filters, or cigarette filters based on a mixture thereof and in an air filter fabric, in particular in a mask.

8. A method of preparing a cigarette filter capable of capturing free radicals, in particular free radicals present in smoke, consisting in:
mixing microparticles formed from a mixture of 2 to 4 saturated vegetable oils, whose melting temperatures differ by at least 10° C, with polyphenolic compounds or their derivatives, in particular in the form of a purified rosemary extract, and
incorporate these microparticles in a filter for tobacco cigarettes, filter for CBD cigarettes, filter for cannabis cigarettes or filter for cigarettes with a mixture density of 0.3 mg/mm3 to 1 mg/mm3.

9. Cigarette filter obtained by the process according to claim 8 wherein said microparticles have a size between 70-200 microns and the filter has a pressure drop of less than 1200 mm of water.

10. Process for preparing a filter fabric capable of capturing free radicals, in particular free radicals present in smoke and polluted air, consisting of:
mixing microparticles formed from a mixture of 2 to 4 saturated vegetable oils, whose melting temperatures differ by at least 10° C, with polyphenolic compounds or their derivatives, in particular in the form of a purified rosemary extract, and
deposit these microparticles on the surface of a filter fabric with a density of 15 gsm to 80 gsm.

11. Filter fabric obtained by the process according to claim 10 wherein said microparticles have a size of between 1 and 20 microns and a draft resistance of less than 150% of the FFP2 or N95 or FFP3 standard.
